**Europäisches Patentamt**

**European Patent Office**

**Office européen des brevets**

(11) Veröffentlichungsnummer : **0 470 044 A1**

(12) # EUROPÄISCHE PATENTANMELDUNG

(21) Anmeldenummer : **91810522.2**

(22) Anmeldetag : **04.07.91**

(51) Int. Cl.⁵ : **A61B 5/087**

(30) Priorität : **05.07.90 CH 2236/90**

(43) Veröffentlichungstag der Anmeldung :
**05.02.92 Patentblatt 92/06**

(84) Benannte Vertragsstaaten :
**AT CH DE DK FR GB IT LI SE**

(71) Anmelder : **OERTLI INSTRUMENTE AG**
**Gemperenstrasse 18**
**CH-9442 Berneck (CH)**

(72) Erfinder : **OERTLI INSTRUMENTE AG**
**Gemperenstrasse 18**
**CH-9442 Berneck (CH)**

(74) Vertreter : **Schick, Carl et al**
**PATENTANWALTS-BUREAU ISLER AG**
**Postfach 6940**
**CH-8023 Zürich (CH)**

(54) **Vorrichtung für spirometrische Zwecke.**

(57) Die Vorrichtung für spirometrische Zwecke weist einen aus einem Durchflusswiderstand (3) und einem Differenzdruckmesser (4) bestehenden Durchflussmesser (3, 4) sowie eine Maske (10) auf. Der Durchflusswiderstand (3) befindet sich im Bereich des einen Endes eines Rohres (1), an dessen anderes Ende ein Luftsauger (2) angeschlossen ist. Das Rohr (1) weist einen Rohranschluss (9) für die Maske (10) auf.

Fig. 1

Die vorliegende Erfindung betrifft eine Vorrichtung für spirometrische Zwecke gemäss dem Oberbegriff des Patentanspruchs 1.

Zur Messung oder Aufzeichnung der Atemluftbewegungen und eines Teils der Atemvolumina werden Spirometer und Pneumotachometer eingesetzt, an die der Proband mit Hilfe eines Mundstückes angeschlossen wird. Solche Vorrichtungen erweisen sich als nachteilig im Hinblick darauf, dass eine im Mundstück vorhandene Messvorrichtung dem Durchgang der Atemluft einen relativ hohen Widerstand entgegenstellt, was eine Verfälschung der Ergebnisse im Vergleich zur natürlichen Atmung mit sich bringt.

Es ist daher Aufgabe der vorliegenden Erfindung, eine Vorrichtung für spirometrische Zwecke der eingangs erwähnten Art zu schaffen, die einen möglichst kleinen Widerstand bei der Bewegung der Atemluft leistet.

Diese Aufgabe wird erfindungsgemäss durch eine Vorrichtung mit den im kennzeichnenden Teil des Patentanspruchs 1 angegebenen Merkmalen gelöst. Weitere vorteilhafte Ausgestaltungen der Erfindung sind in den abhängigen Ansprüchen angegeben.

Im folgenden wird die Erfindung beispielsweise anhand einer Zeichnung näher erläutert. Es zeigen:

Fig. 1 eine schematische Darstellung einer Vorrichtung nach der Erfindung,

Fig. 2 eine schematische Darstellung einer Anordnung mit einer Pumpe zur Eichung der Vorrichtung nach der Erfindung,

Fig. 3 eine schematische Darstellung einer Variante zur Steuerung der Pumpe nach Fig. 2,

Fig. 4 eine schematische Darstellung einer Anordnung zur Messung ergonomischer Daten mit Hilfe der Vorrichtung nach der vorliegenden Erfindung,

Fig. 5 und Fig. 6 Kurven der gemessenen Werte der Druckdifferenzen in Funktion der Zeit bei einem normal atmenden Sportler bzw. bei einer angeschlossenen Pumpe.

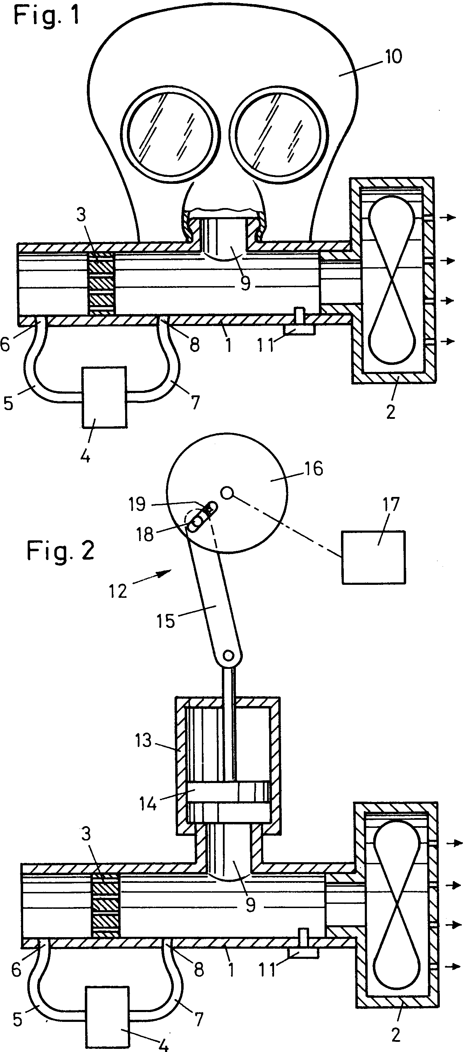

Die Vorrichtung nach Fig. 1 weist ein vorzugsweise flexibles Rohr 1 auf, an dessen ein Ende ein Luftsauger 2 angeschlossen ist. Die Länge des Rohres 1 ist relativ unkritisch. Ein solches Rohr kann beispielsweise ohne weiteres bis 3 m lang sein. Im Bereich des anderen Endes des Rohres 1 ist ein Durchflusswiderstand 3 vorhanden, der beispielsweise ein poröses Membran oder eine Scheibe mit einem Maschengewebe oder eine durchlöcherte Scheibe sein kann. Die Vorrichtung umfasst auch einen Differenzdruckmesser 4, der über einen ersten Schlauch 5 und eine erste Bohrung 6 im Rohr 1 mit dem Innenraum des Rohres 1 vor dem Widerstand 3 und über einen zweiten Schlauch 7 und eine zweite Bohrung 8 im Rohr 1 mit dem Innenraum des Rohres 1 nach dem Widerstand 3 kommuniziert, so dass eine Messung der Druckdifferenz in den Bereichen vor und nach dem Widerstand 3 möglich ist. Der Differenzdruckmesser 4 kann beispielsweise ein piezoelektrisches Element aufweisen.

Im mittleren Bereich des Rohres 1 ist ein Rohranschluss 9 vorhanden, an den ein Atemanschluss oder eine konventionelle Maske 10 angeschlossen werden kann, wobei für den Zweck der vorliegenden Erfindung die Maske 10 ohne Filter und ohne irgendwelche Elemente versehen ist, die einen Widerstand zur Bewegung der Atemluft leisten könnten.

In einem Bereich in der Nähe des Luftsaugers 2 weist das Rohr 1 einen Anschluss 11 auf, an den ein Gasanalysator angeschlossen werden kann. Sonst ist dieser Anschluss geschlossen.

Die Vorrichtung nach Fig. 1 funktioniert folgendermassen:

Der Proband, beispielsweise ein Sportler, zieht sich die Maske 10 derart an, dass er nur durch den Rohranschluss 9 atmen kann. Die Maske 10 wird entweder direkt oder über einen kurzen Schlauch an den Rohranschluss 9 angeschlossen, so dass es sich an dieser Stelle nur ein kleiner toter Raum ergibt. Der Luftsauger 2 saugt die Luft vom Innenraum des Rohres I weg, so dass durch den atmosphärischen Druck Luft durch die Poren oder Löcher des Widerstandes 3 in das Rohr eindringt. Dadurch entsteht eine Druckdifferenz an beiden Seiten des Widerstandes 3, die durch den Differenzdruckmesser 4 gemessen wird. Wenn der Proband nicht atmet oder wenn bei Entfernung der Maske der Anschluss 9 geschlossen ist, misst der Differenzdruckmesser eine konstante Druckdifferenz an beiden Seiten des Widerstandes 3. Wenn der Proband atmet, überlagert sich die Druckdifferenz, die sich bei der Inspiration und Exspiration ergibt. Der Druck im Innenraum des Rohres 1 ist zwar etwas kleiner als der atmosphärische Druck, wie wenn der Proband sich auf einem Berg befinden würde, aber er kann ganz normal atmen, das heisst, ohne einen Luftwiderstand vor dem Mund zu haben, wie es bei anderen Geräten der Fall ist.

Vorzugsweise ist der Luftsauger derart ausgelegt, dass auch im Extremfall bei der Exspiration der Luftdruck im Bereich der Oeffnung 8 kleiner als der Luftdruck im Bereich der Oeffnung 6 ist. Durch Versuche mit einer Vorrichtung nach der Erfindung hat sich ergeben, dass bei Sportlern ein Durchfluss oder eine Luftsaugleistung von ca. 400 Liter pro Minute genügt; vorzugsweise kann dann das Rohr einen Durchmesser von etwa 55 mm aufweisen. Bei Rennpferden wäre die Luftsaugleistung etwa 3000 Liter pro Minute und der Rohrdurchmesser etwa 250 mm. Der Druckmesser misst dann der Verlauf der Druckdifferenz in Funktion der Zeit, während der Proband atmet.

In der Vorrichtung nach Fig. 2 ist der Rohranschluss 9 mit einer Pumpe 12 verbunden, die einen Zylinder 13 und einen Kolben 14 aufweist, der über einen Pleuel 15 gelenkig mit einem Kurbelrad 16 verbunden ist, wobei eine Steuerung 17 zum Antrieb des Kurbelrads 16 dient. Der Pleuel 15 kann an verschiedenen Punkten beispielsweise 18 oder 19 in Fig. 2 angreifen, um einen mehr oder weniger langen Kolbenhub einstellen zu können. Durch die Steuerung 17 kann der Kolben 14 derart bewegt werden, dass es sich in den Bereichen vor und hinter dem Widerstand 3 Druckdifferenzen ergeben, die zur Eichung der Vorrichtung dienen.

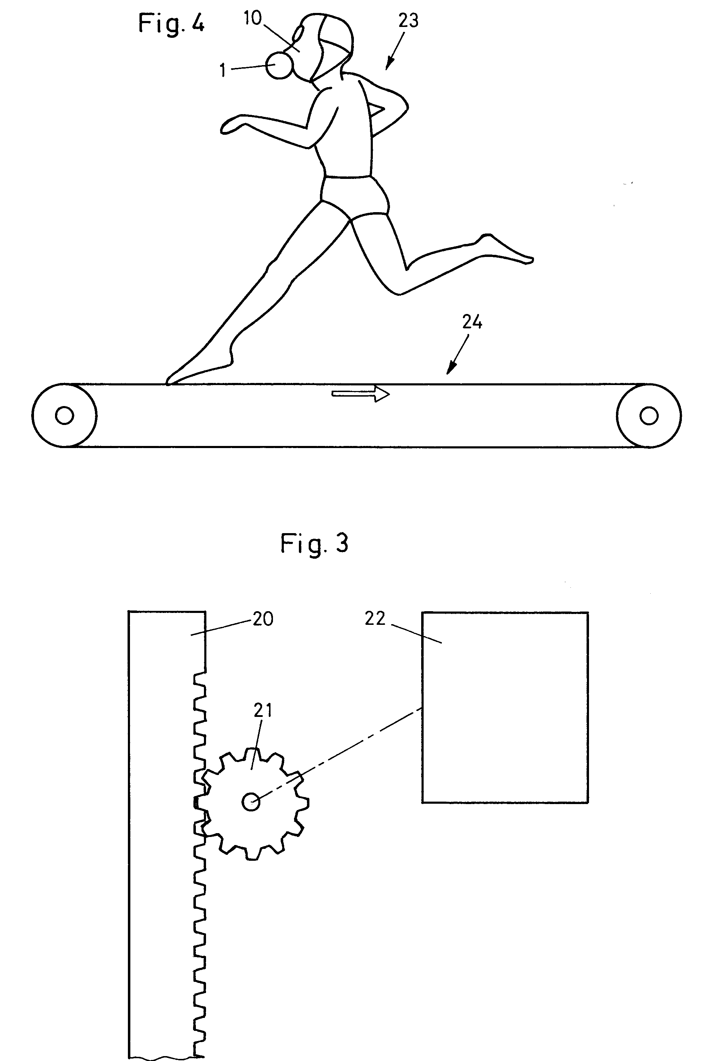

Der Antrieb des Kolbens kann gemäss Fig. 3 auch über eine Zahnstange 20 und ein Ritzel 21 erfolgen, das durch eine Steuerung 22 derart gesteuert wird, dass ein beliebiger Atemvorgang nachgeahmt werden kann.

Wie in Fig. 4 dargestellt, kann ein Sportler 23 oder ein Rennpferd auch derart mit Hilfe der Maske 10 an das Rohr 1 angeschlossen sein, dass er ständig auf einem endlosen Laufband 24 läuft, und zwar in entgegengesetzter Richtung und mit derselben Geschwindigkeit des Laufbands 24, ohne sich von der Stelle zu entfernen. Dabei können bei Verwendung geeigneter Mittel auch verschiedene andere medizinisch relevante Parameter des Probanden, wie Blutdruck, Pulsfrequenz, $O_2$-Verbrauch, $CO_2$-Abgabe usw. gemessen und gegebenenfalls registriert werden.

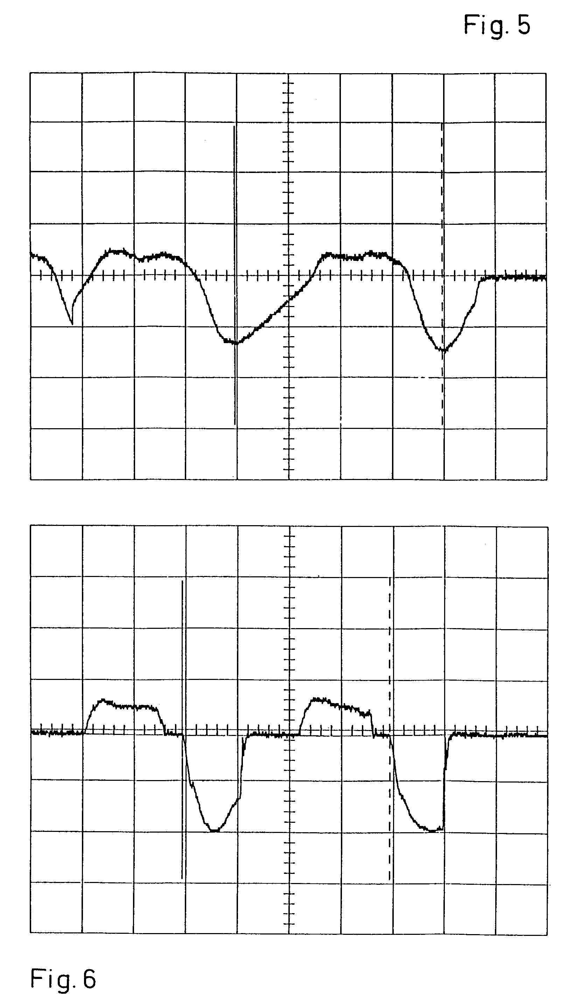

Fig. 5 und Fig. 6 zeigen Beispiele der gemessenen Werte der Druckdifferenzen in Funktion der Zeit bei einem normal atmenden Menschen bzw. bei einer angeschlossenen Pumpe.

Die Saugleistung des Luftsaugers kann derart eingestellt werden, dass die Nullinie des Diagramms zentriert ist. Durch Integration der Druckdifferenzen können bei geeigneter Eichung die Atmungsvolumina mittels einer Korrelationsanalyse ermittelt werden.

Der Proband wird somit mittels der Maske 10 mit einem konstanten Querstrom verbunden, welcher vom Luftsauger 2 erzeugt wird, wobei im Bereich des Anschlusses 11 der Luftstrom im Rohr 1 streng konstant bleibt. Bei einer Inspiration ist an der Stelle des Durchflusswiderstandes 3 eine Flusserhöhung und bei einer Exspiration eine Flussverminderung zu messen. Die Durchflusskurven werden integriert und mit Hilfe einer Simulationslunge geeicht. Die Nullinie entspricht in einem Beispiel einem Querstrom von 400 l/min. Verhält sich der benutzte Flusssensor linear zur Flussdifferenz, so ist auch die Beziehung Fluss/Volumina streng linear. Für die Durchflussmessung können verschiedene Messmethoden wie Differenzmessung (nach der bekannten Methode von A. Fleisch), Thermoelementmessung, Flügelmessgerät, Vartex-Shedding oder Ultraschall-Messsonden verwendet werden.

Wegen der einfachen Kalibrierung und der Konstanz der Messbedingungen erweist sich die erfindungsgemäse Vorrichtung als besonders vorteilhaft in der Leistungsphysiologie und Ergometrie. Diese Vorrichtung braucht zudem wenig Service und ist sehr hygienisch, was Infektionsgefahren von vornherein vermindert. Unter dem Begriff Rohr subsumiert die Erfindung allgemein auch einen länglichen Kasten.

**Patentansprüche**

1. Vorrichtung für spirometrische Zwecke mit einem Durchflussmesser und mit einem Atemanschluss, dadurch gekennzeichnet, dass sich der Durchflussmesser (3, 4) im Bereich des einen Endes eines Rohres (1) befindet, an dessen anderes Ende ein Luftsauger (2) angeschlossen ist, und dass das Rohr (1) einen Rohranschluss (9) für den Atemanschluss (10) aufweist.

2. Vorrichtung nach Anspruch 1, dadurch gekennzeichnet, dass an den Rohranschluss (9) eine Pumpe (13) anschliessbar ist.

3. Vorrichtung nach Anspruch 2, dadurch gekennzeichnet, dass die Pumpe (13) eine Kolbenpumpe (13) mit einem definierten Zylindervolumen ist.

4. Vorrichtung nach Anspruch 3, dadurch gekennzeichnet, dass dieses Zylindervolumen einstellbar ist.

5. Vorrichtung nach einem der Ansprüche 1 bis 4, dadurch gekennzeichnet, dass das Rohr (1) zwei Rohranschlüsse (9) aufweist, einen für den Atemanschluss (10) und den anderen für die Pumpe (13).

6. Vorrichtung nach einem der Ansprüche 2 bis 5, dadurch gekennzeichnet, dass die Pumpe (13) durch eine Steuerung (17) gesteuert ist.

7. Vorrichtung nach Anspruch 6, dadurch gekennzeichnet, dass die Steuerung (17) Aenderungen des totalen Pumpenvolumens bewirkt.

8. Vorrichtung nach einem der Ansprüche 1 bis 7, dadurch gekennzeichnet, dass das Rohr (1) im Bereich nahe dem Luftsauger (2) einen Anschluss (11) für einen Gasanalysator aufweist.

9. Messplatz für physiologische Zwecke mit einer Vorrichtung nach einem der Ansprüche 1 bis 8, dadurch gekennzeichnet, dass Mittel vorhanden sind, um mindestens einen anderen medizinisch relevanten Parameter eines Probanden zu messen.

10. Messplatz für physiologische Zwecke mit einer Vorrichtung nach einem der Ansprüche 1 bis 8 oder nach Anspruch 9, dadurch gekennzeichnet, dass ein endloses Laufband (24) vorhanden ist, auf dem ein Proband in entgegengesetzter Richtung und mit derselben Geschwindigkeit des Laufbands (24) laufen kann, ohne sich vom Messplatz zu entfernen.

Fig. 1

10

3

9

6
5
4
7
8
1
11
2

Fig. 2

16
19
18
17
12
15

13
14

3
9
6
5
4
7
8
1
11
2

Fig. 4

Fig. 3

Fig. 5

Fig. 6

EP 0 470 044 A1

Europäisches Patentamt

# EUROPÄISCHER RECHERCHENBERICHT

Nummer der Anmeldung

EP    91 81 0522

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-2 035 982 (DR. FENYES & GUT)<br>* das ganze Dokument * <br>--- | 1-8 | A61B5/087 |
| A | DE-A-3 844 455 (INSTRUMENTARIUM CORP.)<br>* das ganze Dokument *<br>--- | 1,8 | |
| A | US-A-3 401 683 (P.WEBB ET AL.)<br>* Spalte 2, Zeile 68 - Spalte 4, Zeile 38;<br>Abbildungen 1-3,6 *<br>--- | 1,9,10 | |
| A | US-A-4 463 764 (S.T.ANDERSON ET AL.)<br>* Zusammenfassung; Abbildungen 1,2 *<br>--- | 1,9,10 | |
| A | US-A-3 726 271 (W.G.MONDSHINE ET AL.)<br>* das ganze Dokument *<br>----- | 1 | |
| | | | **RECHERCHIERTE SACHGEBIETE (Int. Cl.5)**<br><br>A61B |

Der vorliegende Recherchenbericht wurde für alle Patentansprüche erstellt

| Recherchenort | Abschlußdatum der Recherche | Prüfer |
|---|---|---|
| DEN HAAG | 30 OKTOBER 1991 | HUNT B.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

8